# EUROPÄISCHE PATENTSCHRIFT

(11) **EP 2 155 638 B1**
(45) Veröffentlichungstag und Bekanntmachung des Hinweises auf die Patenterteilung: **26.11.2014**
(21) Anmeldenummer: 08760702.4
(22) Anmeldetag: 09.06.2008
(51) Int. Cl.: C07C 29/76, C07C 35/12, B01J 2/24

(54) **MENTHOLSCHUPPEN UND VERFAHREN ZU DEREN HERSTELLUNG**
MENTHOL FLAKES AND METHOD FOR PRODUCING THE SAME
FLOCONS DE MENTHOL ET PROCÉDÉ DE FABRICATION DE CEUX-CI

(30) Priorität: 12.06.2007 EP 07110117
(43) Veröffentlichungstag der Anmeldung: 24.02.2010
(73) Patentinhaber: BASF SE, 67056 Ludwigshafen (DE)
(72) Erfinder: RAULS, Matthias, 66440 Blieskastel (DE); BAYER, Robert, 74889 Sinsheim (DE); HEYDRICH, Gunnar, 67117 Limburgerhof (DE); FRYE, Lars, 40764 Langenfeld (DE); WISNIEWSKI, Thomas, 64625 Bensheim (DE)
(86) Internationale Anmeldenummer: PCT/EP2008/057133
(87) Internationale Veröffentlichungsnummer: WO 2008/152009

(56) Entgegenhaltungen:
- DE-A1- 10 224 087
- JP-A- 2004 121 903
- US-A- 3 023 253
- US-A- 3 064 311

## Beschreibung

Die vorliegende Erfindung betrifft ein Verfahren zur Herstellung von L-Menthol in Form fester Schuppen, durch Inkontaktbringen einer L-Menthol-Schmelze mit zwei voneinander beabstandeten gekühlten Oberflächen gemäß Anspruch 1. Darüber hinaus betrifft die vorliegende Erfindung das nach dem genannten Verfahren erhältliche L-Menthol in Form von Schuppen, sowie dessen Verwendung zur Einarbeitung in Gebrauchs- und Verbrauchsgüter aller Art.

Menthol ist ein natürlich vorkommender Wirkstoff, der in Pharmazie, Kosmetik und Lebensmittelindustrie breite Anwendung findet. In natürlichen Quellen, beispielsweise dem Pfefferminzöl, kommt Menthol in Form von vier diastereomeren Enantiomerenpaaren vor, von denen nur die Hauptkomponente, das (-)-Menthol oder L-Menthol die gewünschten geschmacklichen und sonstigen sensorischen Eigenschaften hat.

Von L-Menthol ist seit langem bekannt, dass es in vier verschiedenen Kristallmodifikationen erstarren kann, die bei gleicher chemischer Zusammensetzung unterschiedliche physikalische Eigenschaften aufweisen, wie bereits in J. Am. Chem. Soc., Vol. 39 (8), 1917, S. 1515 bis 1525 beschrieben. So liegen insbesondere die Schmelzpunkte dieser verschiedenen Modifikationen zwischen 33°C bis 43°C wie in Archiv der Pharmazie, 307 (7), 1974, S. 497 bis 503 beschrieben. Der Schmelzpunkt der stabilen alpha-Modifikation liegt demgemäß bei 42 bis 43°C.

Wegen dieser Lage der Schmelzpunkte kann L-Menthol an den Endverbraucher sowohl als in beheizten Behältern flüssig gehaltene Schmelze als auch in Form von Kristallen oder anderen erstarrten Formkörpern abgegeben werden. Generell weisen alle Feststoffe, die, wie L-Menthol, einen Schmelzpunkt nur knapp oberhalb der Umgebungstemperatur haben, eine starke Neigung auf zu verbacken und zu verklumpen. Die Verarbeitung solchermaßen verbackenen Materials ist aber mit erheblichem, unerwünschten Zusatzaufwand verbunden. Soll nun reines L-Menthol, d.h. nicht mit Hilfsmitteln wie z.B. Trennmitteln behandeltes Menthol als Feststoff verkauft werden, muss entweder durch eine geschlossene Kühlkette oder durch die Art der Formgebung sichergestellt werden, dass das Produkt den Endverbraucher in rieselfähiger Form erreicht.

Kommerziell ist Menthol in Form großer Kristalle verfügbar, die bei einer Länge von 0,5 bis 3 cm eine Dicke von 1 bis 3 mm aufweisen. Sie werden traditionell in kleinen Mengen aus natürlich gewonnenem Pfefferminzöl gezüchtet, in dem das Öl in Trögen oder Wannen über viele Tage in Kühlhäusern zur Kristallisation gebracht wird. Diese Kristalle weisen nur bei geringer Schütthöhe eine gute Rieselfähigkeit auf, verbacken aber zusehends bei erhöhter Last und/oder erhöhter Temperatur. Der technische Aufwand für die Kristallisation, Abtrennung und Reinigung der Kristalle und die geringe RaumZeit-Ausbeute eines solchen langwierigen Verfahrens machen es für die großtechnische Anwendung unattraktiv.

Die DE 25 30 481 betrifft eine Vorrichtung zum Kristallisieren von Substanzen, insbesondere von optisch aktiven Mentholen, die unter Kristallisationsbedingungen grobe nadel- und balkenförmige Kristalle bilden. Das diskontinuierlich durchzuführende Kristallisationsverfahren wird unter Einsatz eines besonderen Rührwerkes durchgeführt, das ein Zusammenbacken der Kristalle in der Kristallsuspension verhindert. Das Wertprodukt wird abschließend durch eine Zentrifuge isoliert und in einem Trockner getrocknet.

Die US 3,023,253 und die US 3,064,311 beschreiben geschupptes L-Menthol sowie ein Verfahren zur Herstellung derartiger Schuppen durch Aufbringen einer Schmelze von L-Menthol auf einer gekühlten Tauchwalze. Falls gewünscht kann die Menthol-schmelze zwischen ein Paar gegenläufig rotierender, gekühlter Walzen eingebracht werden. Der auf der Tauchwalze ankristallisierte Mentholfilm wird nachbehandelt, indem er durch Wärmeeintrag getempert und durch Aufbringen zusätzlichen Menthols verstärkt wird. Beide Nachbehandlungen werden simultan mit einer Auftragswalze erreicht. Die so erhaltenen Schuppen weisen zunächst eine gute Rieselfähigkeit auf. Nach längerer Lagerung tritt jedoch eine leichte Verbackung ein, die eine mechanische Auflockerung durch Rütteln des Containers erforderlich macht. Es wird bemerkt, dass diese Verbackung durch eine zwar erwähnte, aber nicht näher charakterisierte poröse Oberfläche und damit einhergehende starke Sublimation des Produkts verursacht wird und dass das so erhaltene Produkt durch Kompaktieren zu Pellets weiterverarbeitet werden kann.

JP 2004121903 beschreibt eine Vorrichtung zur Herstellung von erstarrten Schmelzen in Form von Schuppen, wobei eine flüssige Schmelze auf einer horizontal rotierenden Platte erstarrt, und die erstarrte Schmelze durch eine spezielle Vorrichtung von der rotierenden Platte abgehoben und direkt weiter zu Schuppen zerkleinert wird.

Das Prinzip der weiteren Vergröberung der Primärpartikel durch Kompaktierung wird auch in der DE 102 24 087 betreffend kompaktiertes Menthol in Form von Menthol-Presslingen sowie ein Verfahren zur Herstellung derselben beschrieben. Hier wird aber nicht auf die Wirkung der Partikelgröße allein abgehoben, sondern darauf, dass die Primärpartikel in einer spezifischen Kristallmodifikation vorliegen müssen. Durch Verpressen von Kristallen, die aus einer Lösungskristallisation oder einer Kühlwalzenverschuppung gewonnen wurden, lassen sich gegen Verbackung stabile Kompaktate erhalten, wenn diese überwiegend aus der thermodynamisch stabilen, erst bei 42,5°C schmelzenden alpha-Modifikation bestehen.

In Anbetracht des genannten Standes der Technik bestand die der vorliegenden Erfindung zu Grund liegende Aufgabe in der Bereitstellung eines Verfahren zur Herstellung von verfestigtem Menthol, das folgende vorteilhafte Eigenschaften aufweist: Das Verfahren soll sich insbesondere in technischem Maßstab mit möglichst geringem apparativem Aufwand und hohem Durchsatz möglichst kontinuierlich betreiben lassen; in einem Schritt verfestigtes Menthol zur Verfügung stellen, wobei das erhaltene Menthol in rieselfähiger und über einen längeren Zeitraum nur in geringem Ausmaß zur Verbackung neigenden Form und vorwiegend in der alpha-Modifikation erhalten wird. Daneben soll sich das Verfahren besonders wirtschaftlich, d.h. kostengünstig betreiben lassen.

Beschreibung der Erfindung sowie der bevorzugten Ausführungsformen:
Die Aufgabe wurde überraschend gelöst durch die Bereitstellung eines Verfahrens zur Herstellung von L-Menthol in Form fester Schuppen durch Inkontaktbringen einer L-Menthol-Schmelze mit zwei voneinander beabstandeten gekühlten Oberflächen unter Erstarrung der L-Menthol-Schmelze zu L-Menthol in fester Form, wobei man den Kontakt zwischen der erstarrenden L-Menthol-Schmelze und den gekühlten Oberflächen mindestens bis zum Abschluss der Erstarrung aufrecht erhält und wobei die beiden gekühlten Oberflächen eine planparallele Ausrichtung mit einem Abstand von 0,2 bis 3 mm zueinander aufweisen und unabhängig voneinander jeweils eine Temperatur im Bereich von 0 bis 35°C aufweisen, und Entfernen des erhaltenen L-Menthols in fester Form von einer oder beiden der gekühlten Oberflächen in Form fester Schuppen.

Als Ausgangsstoff zur Durchführung des erfindungsgemäßen Verfahrens eignen sich Schmelzen von L-Menthol der Formel (I), 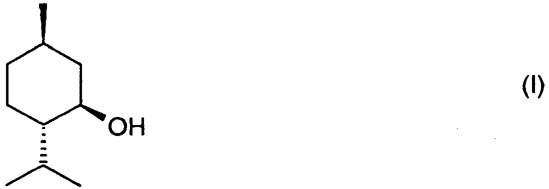 wobei das geschmolzene Menthol natürlichen oder synthetischen Ursprungs sein kann und einen Enantiomerenüberschuss von üblicherweise mindestens 95, 96 oder 97% ee bis 100% ee, bevorzugt 98, 98,5 oder 99 bis 99,9 % ee aufweist. Insbesondere eignen sich als Ausgangsstoffe im Rahmen des erfindungsgemäßen Verfahrens solche Schmelzen von L-Menthol, die einen Gehalt an L-Menthol von mindestens 95, 96 oder 97 Gew.-% oder darüber, bevorzugt mindestens 98 bis 100 Gew.-% und ganz besonders bevorzugt 98, 98,5 oder 99 bis 99,9 Gew.-% aufweisen (jeweils bezogen auf das Gesamtgewicht der Schmelze), neben Verunreinigungen wie beispielsweise Resten von Lösemitteln, Diastereomeren des L-Menthols der Formel (I) oder Nebenprodukten aus Synthese- bzw. Isolierverfahren.

Dabei ist unter dem Begriff L-Menthol-Schmelze vorzugsweise solches L-Menthol zu verstehen, das weitgehend, d.h. zu mindestens 80 oder besser 85 Gew.-%, bevorzugt zu mindestens 90 oder 95 Gew.-% und ganz besonders bevorzugt zu mindestens 95, 96, 97, 98 oder 99 Gew.-% in geschmolzener Form vorliegt, wobei die restlichen Gewichtanteile die Menge an festem L-Menthol in der Schmelze ausmachen. Dabei kann es sich bei dem gegebenenfalls enthaltenen Anteil an festem Menthol in der Schmelze um solches handeln, dass durch einen noch nicht vollständig abgeschlossenen Schmelzprozess des zur Bereitstellung der Schmelze eingesetzten Material noch in der Schmelze vorliegt oder dem vollständig bzw. teilweise geschmolzenen Menthol in fester Form zugesetzt wird, beispielsweise in Form von Kristallen von L-Menthol in der alpha-Modifikation. Solche auch als Impfkristalle zu bezeichnenden Kristalle von L-Menthol in der alpha-Modifikation können beispielsweise auf herkömmliche Weise durch Kristallisation von L-Menthol aus einer L-Menthol-haltigen Lösung oder Schmelze gewonnen werden.

Im Rahmen einer bevorzugten Ausführungsform setzt man solche Kristalle von L-Menthol in der alpha-Modifikation ein, die durch Behandlung der erfindungsgemäß einzusetzenden L-Menthol Schmelze in einem Kratzkühler gewonnen werden, wobei die Impfkristalle in situ in der zu verfestigenden L-Menthol Schmelze gebildet werden, wodurch ein zusätzlicher Arbeitsschritt vermieden wird. Derartige Kratzkühler sind dem Fachmann bekannt und beispielsweise in G. Arkenbout, Melt Crystallization Technology, Technomic Publishing Co. 1995, S. 230 beschrieben.

Die zu verfestigende L-Menthol Schmelze wird, falls gewünscht, in der Regel mit einer möglichst geringen Mengen der genannten Impfkristalle von L-Menthol in der alpha-Modifikation versetzt, entweder, wie vorstehend beschrieben, durch Zusetzen der Kristalle zu der Schmelze oder durch Erzeugung der Kristalle in der Schmelze. Üblicherweise wird die einzusetzende Schmelze, falls gewünscht, mit den genannten Impfkristallen in einer Menge von 0,1 bis 10 Gew.-%, besonders bevorzugt in einer Menge von 0,1 bis 5 Gew.-%, ganz besonders bevorzugt in einer Menge von 0,1 bis 2 Gew.-%, insbesondere in einer Menge von 0,1 bis 1 Gew.-% (jeweils bezogen auf die Gesamtmenge des einzusetzenden Gemisches aus Schmelze und Impfkristallen) versetzt.

Die erfindungsgemäß einzusetzende L-Menthol-Schmelze wird im Rahmen des erfindungsgemäßen Verfahrens üblicherweise bei einer Temperatur im Bereich von etwa 40 bis 60°C, bevorzugt etwa 43 bis 50°C eingesetzt. Dabei handelt es sich bei L-Menthol-Schmelzen im Temperaturbereich von unterhalb von 42 bis 43°C, d.h. unterhalb des Schmelzpunktes von L-Menthol, um unterkühlte Schmelzen.

Die eingesetzte L-Menthol-Schmelze wird erfindungsgemäß mit zwei voneinander beabstandeten, gekühlten Oberflächen in Kontakt gebracht. Bevorzugt befindet sich die eingesetzte L-Menthol-Schmelze im Zwischenraum zwischen den beiden beabstandeten, gekühlten Oberflächen. Das Inkontaktbringen der Schmelze mit den einzelnen Oberflächen kann simultan, d.h. gleichzeitig erfolgen oder zeitlich versetzt erfolgen. Üblicherweise, bedingt durch verfahrenstechnische Erfordernisse, erfolgt das Inkontaktbringen der eingesetzten L-Menthol-Schmelze mit den beiden gekühlten Oberflächen zeitlich versetzt, so dass die Schmelze zunächst mit einer und kurze Zeit darauf zusätzlich mit der zweiten gekühlten Oberfläche in Kontakt kommt. Es hat sich dabei als vorteilhaft erwiesen, die Zeitspanne zwischen dem Inkontaktbringen der L-Menthol-Schmelze mit den jeweiligen gekühlten Oberflächen möglichst gering zu halten, so dass, je nach Temperaturdifferenz zwischen eingesetzter L-Menthol-Schmelze und als erstes in Kontakt gebrachter gekühlter Oberfläche, noch keine weitgehende oder vollständige Verfestigung der eingesetzten L-Menthol Schmelze erfolgt ist, bevor der Kontakt mit der zweiten gekühlten Oberfläche hergestellt ist. Üblicherweise beträgt die Zeitspanne zwischen dem Inkontaktbringen der eingesetzten L-Menthol-Schmelze mit den jeweiligen Oberflächen nicht mehr als 30 s, bevorzugt bis zu 20 s und besonders bevorzugt bis zu 10 s.

Im Rahmen einer bevorzugten Ausführungsform des erfindungsgemäßen Verfahrens handelt es sich bei den erfindungsgemäß einzusetzenden gekühlten Oberflächen jeweils um glatte Oberflachen, bevorzugt um ebene Teilbereiche endloser, aus Stahl, anderen Metallen, Kunststoffen oder Kombinationen dieser Materialien gefertigter Bänder. Besonders bevorzugt sind endlose Bänder aus glattem oder poliertem Edelstahl.

Auch die Dauer des Kontakts der eingesetzten Schmelze, bzw. der erstarrenden Schmelze mit den beiden gekühlten Oberflächen, im folgenden als Kontaktzeit bezeichnet, kann für die einzelnen Oberflächen gleich oder unterschiedlich lang sein. Üblicherweise ist die Kontaktzeit der Schmelze mit den jeweiligen gekühlten Oberflächen verschieden lang, da, wie vorstehend dargestellt, das Inkontaktbringen oft zeitlich versetzt erfolgt und in der Regel auch das Ende der Kontaktzeit, d.h. das Beenden des Kontakts der vollständig erstarrten L-Menthol-Schmelze mit der jeweiligen gekühlten Oberfläche zu verschiedenen Zeitpunkten erfolgt. Unabhängig von der zeitlichen Abfolge des Inkontaktbringens der Schmelze mit den einzelnen der beiden gekühlten Oberflächen und dem Ablösen der vollständig erstarrten L-Menthol-Schmelze von den Oberflächen überlappen die Kontaktzeiten bezüglich der einzelnen gekühlten Oberflächen zeitlich, so dass die eingesetzte L-Menthol-Schmelze bzw. die erstarrende L-Menthol-Schmelze über einen wählbaren Zeitraum gleichzeitig mit beiden gekühlten Oberflächen in Kontakt ist.

Erfindungsgemäß erhält man den Kontakt zwischen der erstarrenden L-Menthol-Schmelze und den gekühlten Oberflächen mindestens bis zum Abschluss der Erstarrung aufrecht. Im Rahmen der vorliegenden Erfindung wird die Erstarrung bzw. Kristallisation der eingesetzten L-Menthol-Schmelze vorzugsweise erst dann als abgeschlossen betrachtet, wenn das erhaltene L-Menthol in fester Form zu mindestens etwa 80 Gew.-% oder besser 85 bis 100 Gew.-%, bevorzugt zu 90 bis 100 Gew.-%, bevorzugt zu 95 oder 97 bis 99,5 Gew.-% und ganz besonders bevorzugt zu 98 bis 99 Gew.-% in der alpha-Modifikation vorliegt. Derartiges L-Menthol weist nur noch in geringem Maße Veränderungen durch Umwandlung des Materials in die thermodynamisch stabilste Modifikation auf und verändert sich daher bezüglich seiner Oberflächenbeschaffenheit, wenn überhaupt, nur noch in geringem Umfang. Die jeweils vorliegende Modifikation des erhaltenen verfestigten L-Menthols und somit der Abschluss des Erstarrungsvorgangs kann mit dem Fachmann bekannten Verfahren wie Röntgenbeugung oder Pulverdiffraktometrie ermittelt werden (s. z.B. Joel Bernstein, Polymorphism in Molecular Crystals, Oxford University Press 2002, S. 94-150).

Unter dem Begriff "gekühlte Oberflächen" sind im Rahmen der vorliegenden Erfindung solche Oberflächen zu verstehen, die eine Temperatur unterhalb des Schmelz- bzw. Erstarrungspunktes von L-Menthol von 42 bis 43°C aufweisen bzw. auf eine solche Temperatur temperiert sind. Die erfindungsgemäß einzusetzenden gekühlten Oberflächen weisen unabhängig voneinander jeweils eine Temperatur im Bereich von 0 bis 35°C, besonders bevorzugt 5 bis 30°C und ganz besonders bevorzugt im Bereich von 10 bis 25°C auf. Dabei können beide Oberflächen die gleiche oder eine unterschiedliche Temperatur aufweisen. Es ist auch möglich, die Temperatur der gekühlten Oberflächen, gewünschtenfalls individuell, im Verlauf der jeweiligen Kontaktzeit zu verändern, d.h. anzuheben oder abzusenken.

Im Rahmen des erfindungsgemäßen Verfahrens weisen die beiden gekühlten Oberflächen eine planparallele Ausrichtung mit einem Abstand von 0,2 bis 3 mm, bevorzugt 0,3 bis 3 mm, besonders bevorzugt 0,5 bis 2,5 mm und ganz besonders bevorzugt 0,75 bis 2,0 mm zueinander auf. Unter dem Begriff planparallele Ausrichtung ist dabei zu verstehen, dass die beiden gekühlten Oberflächen im Rahmen üblicher Messgenauigkeiten über den gesamten, mit der zu verfestigenden L-Menthol Schmelze in Kontakt gebrachten Bereich oder Teilbereich den gleichen Abstand aufweisen. Der zwischen den beiden gekühlten Oberflächen gebildete Zwischenraum ist vorteilhafterweise vollständig mit L-Menthol angefüllt, weil so eine möglichst große Kontaktfläche zwischen den gekühlten Oberflächen und der zu verfestigenden L-Menthol-Schmelze gegeben ist.

In Abhängigkeit von den gewählten Temperaturen der eingesetzten L-Menthol-Schmelze sowie den beiden gekühlten Oberflächen wird die Kontaktzeit der erstarrenden L-Menthol-Schmelze mit den beiden gekühlten Oberflächen vorteilhafterweise so gewählt, dass sie die Dauer bis zur Ausbildung der alpha-Modifikation des verfestigten L-Menthols aus den im Rahmen des erfindungsgemäßen Verfahrens wahrscheinlich primär gebildeten Modifikationen wie beispielsweise der gamma-Modifikation nur geringfügig übersteigt. Üblicherweise ist die Erstarrung nach einer Kontaktzeit von etwa 10 bis etwa 300 s, bevorzugt etwa 20 bis etwa 250 s, bevorzugt bis etwa 200 s und ganz besonders bevorzugt von 30 bis 150 s, bevorzugt bis 100 s abgeschlossen, d.h. das erhaltene L-Menthol in fester Form liegt wie vorstehend beschrieben zu mindestens 80 Gew.-% in der alpha-Modifikation vor. Dabei sind die genannten Kontaktzeiten so zu verstehen, dass sie die Zeitspannen angeben, während derer gleichzeitiger Kontakt zwischen der L-Menthol-Schmelze bzw. der erstarrenden oder bereits erstarrten L-Menthol-Schmelze zu beiden gekühlten Oberflächen besteht. Gewünschtenfalls kann der Kontakt der erstarrten L-Menthol-Schmelze mit einer der beiden gekühlten Oberflächen auch darüber hinaus ausgedehnt werden.

Kurze Kontaktzeiten und eine vollständige Erstarrung in der alpha-Modifikation können im Rahmen einer bevorzugten Ausführungsform des erfindungsgemäßen Verfahrens erreicht werden, in dem die Schmelze vor oder während des Inkontaktbringens mit den gekühlten Oberflächen bzw. vor oder während der Aufgabe auf das Kühlband wie vorstehend beschrieben mit Impfkristallen versetzt wird. Dies kann beispielsweise durch Einrühren in ein Vorlagegefäß oder Aufstreuen von zuvor zerkleinerten Kristallen der alpha-Modifikation des L-Menthols auf die eingesetzte L-Menthol-Schmelze (den flüssigen Kristallfilm) erreicht werden. In einer bevorzugten Ausführungsform der Erfindung wird die Impfung durch Passage der Schmelze durch einen unterhalb des Schmelzpunktes betriebenen Wärmetauscher erreicht, dessen Wände durch ein Abriebsorgan von aufkristallisiertem Material befreit werden. Dem Fachmann ist diese Anordnung beispielsweise in der Form eines wie vorstehend genannten Kratzkühlers geläufig. Dementsprechend zeichnet sich eine bevorzugte Ausführungsform des erfindungsgemäßen Verfahrens dadurch aus, dass die Impfkristalle durch Behandlung der einzusetzenden L-Menthol-Schmelze in einem Kratzkühler gebildet werden.

Das erfindungsgemäß erhaltene L-Menthol in fester Form wird dann nach dem Fachmann bekannten Verfahren von der bzw. den gekühlten Oberflächen entfernt. Erfindungsgemäß bevorzugt entfernt man das erstarrte L-Menthol mit einem angestellten Messer von einer oder beiden der gekühlten Oberflächen unter Erhalt von L-Menthol in Form fester Schuppen.

Im Rahmen einer besonders bevorzugten Ausführungsform führt man das erfindungsgemäße Verfahren unter Einsatz eines Doppelbandkühlers durch. Doppelbandkühler sind dem Fachmann bekannt und können beispielsweise von den Firmen Sandvik Process Systems GmbH, D-70736 Fellbach oder Kaiser Steelbelt Systems GmbH, D-47800 Krefeld bezogen werden.

Bei Einsatz der genannten Doppelbandkühler werden die erfindungsgemäß einzusetzenden gekühlten Oberflächen in Form zweier in entgegengesetztem Drehsinn über Walzen geführter, üblicherweise aus Stahl gefertigter Endlosbänder (Kühlbändern) realisiert (s. C.M. van't Land, Industrial Crystallization of Melts, Marcel Dekker 2005, S. 63). Die erfindungsgemäße Erstarrung der L-Menthol-Schmelze zu L-Menthol in fester Form findet dann im Zwischenraum zwischen den planparallel verlaufenden, einander zugewandten Teilbereichen der beiden Kühlbänder des Doppelbandkühlers statt.

Um ein möglichst gleichzeitiges Inkontaktbringen der zu verfestigenden L-Menthol-Schmelze mit den beiden Kühlbändern zu erreichen, empfiehlt es sich, die Schmelze möglichst nah an der Stelle mit den Kühlbändern in Kontakt zu bringen, an der der Zwischenraum zwischen den planparallel verlaufenden Teilbereichen der beiden Kühlbänder beginnt, so dass es in möglichst geringem Ausmaß zu einer vorzeitigen Verfestigung der L-Menthol-Schmelze kommt.

Nach wie vorstehend beschriebener Entfernung des erhaltenen L-Menthols in fester Form durch Schuppung, kann das so erhaltene Material noch durch weitere Kühlung, beispielsweise auf einer gekühlten Förderschnecke oder einem gekühlten Förderband, nachbehandelt werden.

Das erfindungsgemäße Verfahren kann diskontinuierlich, beispielsweise unter Einsatz gekühlter Stempel oder, beispielsweise unter Einsatz eines wie vorstehend genannten Doppelbandkühlers, kontinuierlich durchgeführt werden. Dabei eröffnet insbesondere die kontinuierliche Verfahrensweise wirtschaftliche Vorteile.

Durch das erfindungsgemäße Verfahren ist L-Menthol in Form fester Schuppen erhältlich, die, bedingt durch die Erstarrung im Kontakt mit zwei gekühlten Oberflächen, zumindest zwei glatte Oberflächen aufweisen, wobei es an beiden Oberflächen nicht mehr zur nachträglichen Umwandlung des an der Oberfläche befindlichen L-Menthols von der gamma- in die alpha-Modifikation kommt. Dadurch bleibt die glatte Oberflächenstruktur an den beiden Kontaktflächen zu den gekühlten Oberflächen erhalten, die dem so erhaltenen L-Menthol in Form fester Schuppen vorteilhafte Eigenschaften wie beispielsweise eine glatte, stabile Oberfläche sowie eine geringe Haftungsneigung verleihen, wobei die genannten Materialeigenschaften auch nach längerer Lagerungsdauer von einigen Wochen bei Umgebungstemperatur erhalten bleiben. Die beiden glatten Oberflächen des erfindungsgemäß erhaltenen verfestigten Menthols neigen nicht zur Ausbildung aus der Oberfläche heraus- bzw. durch Desublimation darauf aufwachsender mikroskopischer nadelförmiger Kristalle, die möglicherweise für die Verklumpungsneigung des auf herkömmlich Weise verfestigten L-Menthols verantwortlich sind. Die vorliegende Erfindung betrifft demgemäß in einem weiteren Aspekt L-Menthol in Form von Schuppen, die erhältlich nach dem erfindungsgemäßen, vorstehend beschriebenen Verfahren sind.

Löst man das erfindungsgemäß erhältliche L-Menthol in Form fester Schuppen von einer oder beiden der gekühlten Oberflächen, vorzugsweise den Kühlbändern eines erfindungsgemäß bevorzugt einzusetzenden Doppelbandkühlers ab, erhält man semi-transparente L-Menthol Schuppen, die die dargestellten vorteilhaften Materialeigenschaften aufweisen.

Nach dem erfindungsgemäßen Verfahren erhältliches Menthol in Form von Schuppen weist, je nach gewähltem Abstand der beiden gekühlten Oberflächen, eine gleichmäßige Dicke bzw. Stärke von 0,2 bis 3 mm, bevorzugt 0,3 bis 3 mm, besonders bevorzugt 0,5 bis 2,5 mm, und ganz besonders bevorzugt 0,75 bis 2 mm auf. Der mittlere, auf die glatte Fläche bezogene Durchmesser kann je nach Art der Schuppung bzw. Ablösung von den gekühlten Oberflächen frei gewählt werden und reicht von endlosen Bändern bis hin zu stark zerkleinerten Schuppen. Im Hinblick auf die gute Handhabbarkeit des erhaltenen verfestigten Menthols haben sich Schuppen mit einem mittleren Durchmesser von etwa 1 bis etwa 20 mm, bevorzugt etwa 3 bis etwa 10 mm als vorteilhaft und somit erfindungsgemäß bevorzugt erwiesen.

Das so zugängliche L-Menthol in Form fester Schuppen, eignet sich aufgrund seiner vorteilhaften Materialeigenschaften zur Weiterverarbeitung, beispielsweise zur Einarbeitung in Gebrauchs- oder Verbrauchsgüter wie beispielsweise pharmazeutische oder kosmetische Zubereitungen, Nahrungsmittel, Hygiene- oder Reinigungsartikel, Süßwaren oder Tabakerzeugnisse. Die vorliegende Erfindung betrifft daher in einem weiteren Aspekt die Verwendung des erfindungsgemäß erhältlichen L-Menthols in Form von Schuppen zur Herstellung von oder Einarbeitung in Produkte wie Gebrauchs- oder Verbrauchsgüter.

Die folgenden Beispiele dienen der Erläuterung der Erfindung, ohne sie in irgendeiner Weise zu beschränken:

### Beispiel 1:

Ein Tropfen schmelzflüssigen L-Menthols wurde auf eine auf 25°C gekühlte Stahlplatte gegeben. Der sich ausbildenden Lache verlaufenen Menthols wurde vor dem vollständigen Erstarren ein auf 25°C temperierter Stahlstempel mit einer Last von ca. 2 N/cm² aufgeprägt. Das L-Menthol erstarrte innerhalb von 1 Minute zu einer wachsartigen Masse und war nach 2 bis 3 Minuten so weit kristallisiert, dass es unter Brechen von der Platte abgehoben werden konnte. Man erhielt auf beiden Seiten glänzend-glatte, semitransparente Schuppen.

### Beispiel 2:

Eine auf 50°C temperierte Schmelze von L-Menthol wurde in einen auf 10°C temperierten Kratzkühler eingetragen, und flüssig, aber durch Impfkristalle getrübt ausgetragen und über ein Wehr als flüssiger Film auf ein von beiden Seiten auf 10°C temperiertes Doppelkühlband aufgebracht. Nach 90 s Laufzeit wurde am Ende des Bandes ein 1,5 mm dicker, durchkristallisierter Film von L- Menthol gewonnen, der mit einem Schneidwerk in 4 bis 8 mm große Schuppen zerkleinert wurde. Man erhielt auf beiden Seiten glänzend erscheinende, semi-transparente Schuppen, deren Haufwerk auch nach einer Lagerung von 7 Tagen bei Temperaturen von 25°C noch durch leichtes Schütteln aufgelockert werden konnte. Die Wände der eingesetzten Aufbewahrungsgefäße zeigten dann nur Spuren von Sublimatabscheidung.

### Vergleichsbeispiel 1:

Eine auf 50°C temperierte Schmelze von Menthol wurde dem Tauchbecken einer Schuppenwalze zugeführt. Auf die auf 15°C temperierte Walze wurde ein Film von L-Menthol aufgezogen, der während der Rotation der Walze durch den Luftraum erstarrt. Nach einer Verweilzeit von 60 s wurde eine 0,7 mm starke, noch wachsartige Mentholschicht erhalten, die nach weiteren 2 Minuten Abkühlung an der Luft von einem Schneidwerk in 5 bis10 mm große Schuppen zerkleinert wurde. Diese Schuppen wiesen einseitig eine hoch-poröse, raue Oberfläche auf. Ihr Haufwerk war nach wenigen Tagen bei Umgebungsbedingungen stark verklumpt, die Gefäßwände waren mit großen Mengen Sublimat bedeckt.

### Vergleichsbeispiel 2:

Ein Tropfen schmelzflüssigen L-Menthols wurde auf eine auf 25°C gekühlte Stahlplatte gegeben. Die sich ausbildende Lache verlaufenen Menthols erstarrte innerhalb von 2 Minuten zu einer wachsartigen Masse und war nach 3 bis 4 Minuten so weit kristallisiert, dass es unter Brechen von der Platte abgehoben werden konnte. Man erhielt L-Menthol-Schuppen, die eine ausgeprägt glatte, glänzende Seite und eine schneeweiß leuchtende, raue Oberfläche aufwiesen.

## Patentansprüche

1. Verfahren zur Herstellung von L-Menthol in Form fester Schuppen durch Inkontaktbringen einer L-Menthol-Schmelze mit zwei voneinander beabstandeten gekühlten Oberflächen unter Erstarrung der L-Menthol-Schmelze zu L-Menthol in fester Form, wobei man den Kontakt zwischen der erstarrenden L-Menthol-Schmelze und den gekühlten Oberflächen mindestens bis zum Abschluss der Erstarrung aufrecht erhält und wobei die beiden gekühlten Oberflächen eine planparallele Ausrichtung mit einem Abstand von 0,2 bis 3 mm zueinander aufweisen und unabhängig voneinander jeweils eine Temperatur im Bereich von 0 bis 35°C aufweisen, und Entfernen des erhaltenen L-Menthols in fester Form von einer oder beiden der gekühlten Oberflächen in Form fester Schuppen.

2. Verfahren nach Anspruch 1, **dadurch gekennzeichnet, dass** man den Kontakt zwischen der erstarrenden L-Menthol-Schmelze und den gekühlten Oberflächen so lange aufrecht erhält, bis das L-Menthol in fester Form zu mindestens 80 Gew.% in der alpha-Modifikation vorliegt.

3. Verfahren nach einem der Ansprüche 1 bis 2, **dadurch gekennzeichnet, dass** der Zwischenraum zwischen den beiden gekühlten Oberflächen vollständig mit Menthol angefüllt ist.

4. Verfahren nach einem der Ansprüche 1 bis 3, **dadurch gekennzeichnet, dass** die Kontaktzeit zwischen L-Menthol und den gekühlten Oberflächen 10 bis 300 s beträgt.

5. Verfahren nach einem der Ansprüche 1 bis 4, **dadurch gekennzeichnet, dass** man eine L-Menthol-Schmelze einsetzt, die einen Enantiomerenüberschuss von mindestens 95 % ee und einen Gehalt an L-Menthol von mindestens 95 Gew.-%, bezogen auf das Gesamtgewicht der Schmelze, aufweist

6. Verfahren nach einem der Ansprüche 1 bis 5, **dadurch gekennzeichnet, dass** man eine L-Menthol-Schmelze mit einer Temperatur im Bereich von 40 bis 60°C einsetzt.

7. Verfahren nach einem der Ansprüche 1 bis 6, **dadurch gekennzeichnet, dass** man die einzusetzende L-Menthol-Schmelze vor dem Inkontaktbringen mit den gekühlten Oberflächen mit 0,1 bis 10 Gew.-% Impfkristallen von L-Menthol versetzt.

8. Verfahren nach Anspruch 7, **dadurch gekennzeichnet, dass** die Impfkristalle durch Behandlung der einzusetzenden L-Menthol-Schmelze in einem Kratzkühler gebildet werden.

9. Verfahren nach einem der Ansprüche 1 bis 8, **dadurch gekennzeichnet**, das man es unter Einsatz eines Doppelbandkühlers durchführt.

10. Verfahren nach einem der Ansprüche 1 bis 9, **dadurch gekennzeichnet, dass** die beiden gekühlten Oberflächen die planparallel verlaufenden Teilbereiche der Kühlbänder eines Doppelbandkühlers sind.

11. L-Menthol in Form fester Schuppen, erhältlich nach dem Verfahren gemäß einem der Ansprüche 1 bis 10.

12. L-Menthol-Schuppen nach Anspruch 11 mit einer Dicke von 0,2 bis 3 mm.

13. L-Menthol-Schuppen nach Anspruch 11 oder 12 mit einem mittleren Durchmesser von 1 bis 20 mm.

14. Verwendung von L-Menthol in Form von Schuppen nach einem der Ansprüche 11 bis 13 zur Herstellung von oder Einarbeitung in Gebrauchs- und Verbrauchsgüter.

## Claims

1. A method for preparing L-menthol in the form of solid flakes by bringing an L-menthol melt into contact with two chilled surfaces distanced from one another with solidification of the L-menthol melt to give L-menthol in solid form, where the contact between the solidifying L-menthol melt and the chilled surfaces is maintained at least until completion of the solidification and where the two chilled surfaces have a plane-parallel orientation with a distance of 0.2 to 3 mm relative to one another and, independently of one another each have a temperature in the range from 0 to 35°C, the resulting L-menthol and removing in solid form from one or both of the chilled surfaces in the form of solid flakes.

2. The method according to claim 1, wherein the contact between the solidifying L-menthol melt and the chilled surfaces is maintained until the L-menthol is present in solid form to at least 80% by weight in the alpha modification.

3. The method according to any one of claims 1 to 2, wherein the interspace between the two chilled surfaces is completely filled with menthol.

4. The method according to any one of claims 1 to 3, wherein the contact time between L-menthol and the chilled surfaces is 10 to 300 s.

5. The method according to any one of claims 1 to 4, wherein an L-menthol melt is used which has an enantiomer excess of at least 95% ee and a content of L-menthol of at least 95% by weight, based on the total weight of the melt.

6. The method according to any one of claims 1 to 5, wherein an L-menthol melt with a temperature in the range from 40 to 60°C is used.

7. The method according to any one of claims 1 to 6, wherein the L-menthol melt to be used is treated with 0.1 to 10% by weight of seed crystals of L-menthol before being brought into contact with the chilled surfaces.

8. The method according to claim 7, wherein the seed crystals are formed by treating the L-menthol melt to be used in a scratch chiller.

9. The method according to any one of claims 1 to 8, which is carried out using a twin-belt chiller.

10. The method according to any one of claims 1 to 9, wherein the two chilled surfaces are the plane-parallel sections of the chilling belts of a twin-belt chiller.

11. L-menthol in the form of solid flakes obtainable by the method according to any one of claims 1 to 10.

12. L-menthol flakes according to claim 11 with a thickness of from 0.2 to 3 mm.

13. L-menthol flakes according to claim 11 or 12 with an average diameter of from 1 to 20 mm.

14. The use of L-menthol in the form of flakes according to any one of claims 11 to 13 for the production of or incorporation into utility and consumer goods.

## Revendications

1. Procédé pour la préparation de L-menthol sous forme d'écailles solides par mise en contact d'une masse fondue de L-menthol avec deux surfaces refroidies écartées l'une de l'autre avec solidification de la masse fondue de L-menthol en L-menthol sous forme solide, la mise en contact entre la masse fondue de L-menthol en solidification et les surfaces refroidies étant maintenue au moins jusqu'à la fin de la solidification et les deux surfaces refroidies présentant une orientation à plans parallèles d'une distance de 0,2 à 3 mm l'une de l'autre et présentant, indépendamment l'une de l'autre, à chaque fois une température dans la plage de 0 à 35°C et par élimination du L-menthol obtenu sous forme solide d'une ou des deux surfaces refroidies sous forme d'écailles solides.

2. Procédé selon la revendication 1, **caractérisé en ce qu'**on maintient le contact entre la masse fondue de L-menthol en solidification et les surfaces refroidies jusqu'à ce que le L-menthol sous forme solide se trouve à raison d' au moins 80% en poids dans la modification alpha.

3. Procédé selon l'une quelconque des revendications 1 à 2, **caractérisé en ce que** l'espace intermédiaire entre les deux surfaces refroidies est complètement rempli de menthol.

4. Procédé selon l'une quelconque des revendications 1 à 3, **caractérisé en ce que** le temps de contact entre le L-menthol et les surfaces refroidies est de 10 à 300 s.

5. Procédé selon l'une quelconque des revendications 1 à 4, **caractérisé en ce qu'**on utilise une masse fondue de L-menthol, qui présente un excès d'énantiomère d'au moins 95% d'ee et une teneur en L-menthol d'au moins 95% en poids, par rapport au poids total de la masse fondue.

6. Procédé selon l'une quelconque des revendications 1 à 5, **caractérisé en ce qu'**on utilise une masse fondue de L-menthol à une température dans la plage de 40 à 60°C.

7. Procédé selon l'une quelconque des revendications 1 à 6, **caractérisé en ce qu'**on ajoute à la masse fondue de L-menthol à utiliser, avant la mise en contact avec les surfaces refroidies, 0,1 1 à 10% en poids de cristaux de germes de L-menthol.

8. Procédé selon la revendication 7, **caractérisé en ce que** les cristaux de germes sont formés par traitement de la masse fondue de L-menthol à utiliser dans un refroidisseur à racleur.

9. Procédé selon l'une quelconque des revendications 1 à 8, **caractérisé en ce qu'**on le réalise en utilisant un refroidisseur à double bande.

10. Procédé selon l'une quelconque des revendications 1 à 9, **caractérisé en ce que** les deux surfaces refroidies sont les zones partielles s'étendant en plans parallèles des bandes de refroidissement d'un refroidisseur à double bande.

11. L-menthol sous forme d'écailles solides pouvant être obtenu selon le procédé selon l'une quelconque des revendications 1 à 10.

12. Ecailles de L-menthol selon la revendication 11 présentant une épaisseur de 0,2 à 3 mm.

13. Ecailles de L-menthol selon la revendication 11 ou 12 présentant un diamètre moyen de 1 à 20 mm.

14. Utilisation de L-menthol sous forme d'écailles selon l'une quelconque des revendications 11 à 13 pour la préparation de ou l'incorporation dans des produits d'utilisation et de consommation.
